# EUROPEAN PATENT APPLICATION

(11) **EP 1 721 589 A1**
(43) Date of publication of application: **15.11.2006**
(21) Application number: 05719652.9
(22) Date of filing: 28.02.2005
(51) Int. Cl.: A61F 13/49, A61F 5/44, A61F 13/15, A61F 13/511, A61F 13/53, A61F 13/534

(54) **ABSORBENT AND DIAPERS AND ABSORBENT ARTICLES PROVIDED WITH THE SAME**

(30) Priority: 01.03.2004 JP 2004056646
(71) Applicant: DAIO PAPER CORPORATION, Shikokuchuo-shi, Ehime 799-0402 (JP)
(72) Inventor: MORI, Isao, c/o Daio Paper Converting Co., Ltd.,, Shikokuchuo-shi, Ehime 7990431 (JP); FUKAE, Akinori, c/o Daio Paper Converting Co., Ltd, Shikokuchuo-shi, Ehime 7990431 (JP); HANAO, Hiroyuki c/o Daio Paper Converting Co.,Ltd., Shikokuchuo-shi, Ehime 7990431 (JP); ITO, Kazunori, c/o Daio Paper Converting Co., Ltd., Shikokuchuo-shi, Ehime 7990431 (JP)
(74) Representative: Harris, Ian Richard
(86) International application number: PCT/JP2005/003335
(87) International publication number: WO 2005/082305

(57) **Abstract**

Disclosed is as absorbent assembly including a main absorbent part (21) composed of a highly absorbent polymer and absorbent fibers, a crepe paper sheet (22) wrapped around the main absorbent part, and a second sheet (25) arranged on a portion of the crepe paper sheet (22) wrapped around one of the major surfaces of the main absorbent part (21). The major surfaces of the main absorbent part (21) are hot-melt bonded to the crepe paper sheet (22). The weight ratio of the highly absorbent polymer to the total weight of the highly absorbent polymer and absorbent fibers of the main absorbent part (21) is set to 40 wt% or higher. The absorbent assembly is pressurized to reduce its thickness. The second sheet (25) contains polyethylene terephthalate, exhibiting high cushioning performance, as its component material. The liquid is diffused and the area over which the liquid is intruded into the main absorbent part is enlarged to prevent the absorptive power of the absorbent assembly from being lowered.

## Description

### Technical Field

This invention relates to an absorbent assembly formed by a highly absorbent polymer and absorbent fibers and which has been pressurized and thereby reduced in thickness. The present invention also relates to an absorbent assembly and to an absorbent product including this absorbent assembly.

The present application asserts the Convention rights based on a Japanese Patent Application filed in Japan on March 1, 2004 under the application number of 2004-56646, the entire contents of which are incorporated herein by reference.

### Background Art

For absorbent products, such as disposable paper diaper, physiological napkin or incontinence pad, an absorbent assembly, making use of a highly absorbent polymer, is used.

The highly absorbent polymer, also termed SAP or highly absorbent resin, has characteristics such that it absorbs water and becomes wetted and swollen, on contact with water, to take the form of a gel, and that, once it has absorbed water, does not permit the liquid to exit even on application of a sizeable pressure. The amount of absorption of the highly absorbent polymer amounts to tens to thousands of times as large as the own volume.

Although the highly absorbent polymer by itself exhibits the above-described high absorptive power, this absorptive power may not be exhibited unless the polymer has become wetted and swollen to a more or less extent. Moreover, its rate of water absorption is that low. For this reason, the polymer in the absorbent assembly is mixed in the form of powders, obtained on crushing or pulverization, in absorbent fibers, such as pulp obtained on crushing, or in rayon.

The absorbent assembly, used in the absorbent products, such as physiological napkins, incontinence pads or in diapers, as described above, includes the highly absorbent polymer and absorbent fibers, and hence is bulky and inconvenient to carry about. Since the absorbent assembly is bulky, it does not lend itself to improving the efficiency in goods distribution. For this reason, the absorbent assembly, used for the absorbent products, is press-worked for reducing its volume, in an attempt to reduce the thickness and size of the absorbent product,

However, the absorbent assembly, press-worked for reducing its thickness, is drastically raised in packing density of the absorbent ingredients, such as pulp, obtained on crushing, so that its absorptive power, such as volume or rate of absorption or the liquid transmissive properties, is lowered. Hence, the absorbent assembly, reduced in thickness by the press-working, is usually processed to increase the weight per unit area to maintain its absorptive power. However, this technique is not sufficient to reduce the thickness of the absorbent assembly sufficiently. Moreover, if the highly absorbent polymer is increased excessively in density, as a result of reduction in thickness, the so-called gel blocking is produced so that the desired absorptive power of the polymer may not be developed. The gel blocking is a phenomenon in which the voids between neighboring products of the swollen highly absorbent polymer are reduced drastically such that a desired absorptive power cannot be developed. The result is that urine permeation is obstructed by affixture of neighboring products of the highly absorbent polymer to one another so that the urine that cannot be permeated has its flow reversed to exit the absorbent assembly.

On the other hand, the absorbent assembly, reduced in thickness by the press working, becomes hardened due to increased packing density. Thus, if the assembly is used in an absorbent product which, when in use, contacts the body of a user, he/she may feel stiff. In addition, the product may become appreciably creased, so that there may arise a problem such as leakage of liquid from the absorbent assembly.

The technical literature, as a predecessor of the present application, is the publication of JP Laid-Open Patent Publication 2002-172139,

### Disclosure of the Invention

### Problems to be solved by the Invention

The present invention is proposed in light of the above-described state of the relevant technical field. It is as object of the present invention to provide an absorbent assembly which may be reduced in thickness without lowering its absorptive power, a diaper having the absorbent assembly, and an absorbent product having this absorbent assembly.

An absorbent assembly of the present invention, proposed for accomplishing the above object, includes a main absorbent part composed of a highly absorbent polymer and absorbent fibers, a crepe paper sheet wrapped around the main absorbent part, and a liquid permeable sheet arranged on a portion of the crepe paper sheet wrapped around one of the major surfaces of the main absorbent part. The major surfaces of the main absorbent part are hot-melt bonded to the crepe paper sheet The weight ratio of the highly absorbent polymer to the total weight of the highly absorbent polymer and absorbent fibers of the main absorbent part is set to 40 wt% or higher. The main absorbent part is pressurized and reduced in thickness. The liquid permeable sheet is formed, at least in part, of polyethylene terephthalate, as its component, for improving the cushioning performance and for allowing the liquid to be diffused over a wide area to the main absorbent part. The liquid permeable sheet may further be formed in part of hollow polyethylene terephthalate as its component to further increase the cushioning performance.

The hot-melt bonding between one of the major surfaces of the main absorbent part and the crept paper sheet may be by a delayed crystallization adhesive to enhance the anchoring performance of the highly absorbent polymer. The hot-melt bonding between the other major surface of the main absorbent part and the crepe paper sheet may be by a water-proofing adhesive for anchoring the highly absorbent polymer and the absorbent fibers to each other after liquid absorption.

The surface of the main absorbent part may be formed to be substantially planar and may also be embossed to hold the highly absorbent polymer in place by the absorbent fibers to improve the anchoring performance of the highly absorbent polymer.

For maintaining the absorptive power of the main absorbent part and reducing its thickness, the weight per unit area and the density of the highly absorbent polymer are preferably not less than 300 gsm and not less than 150 kg/m³, respectively. The overall thickness of the main absorbent part and the density of the highly absorbent polymer are preferably 1.0 to 1.8 mm and not less than 300 kg/m³, respectively.

With the above-described absorbent assembly, the main absorbent part, wrapped by the crepe paper sheet, and the liquid permeable sheet, may be wrapped by a sheet, at least the liquid intruding surface of which has been made liquid permeable, and may, in this state, be mounted on an absorbent product. In addition, the absorbent assembly may be mounted as one on a component member of the absorbent product.

That is, with the diaper according to the present invention, the aforementioned absorbent assembly and the liquid permeable sheet, as a second sheet, are mounted between the liquid permeable top sheet and the liquid impermeable back sheet. Also, with the absorptive product, other than the diaper, such as the physiological napkin or the incontinence pad, the aforementioned absorbent assembly and the liquid permeable sheet, as the second sheet, are mounted between the liquid permeable top sheet and the liquid impermeable back sheet Other objects and specified advantages of the present invention will become more apparent from the following explanation of preferred embodiments thereof especially when read in conjunction with me drawings,

### Brief Description of the Drawings

Fig.1 is a developed plan view of a panty type diaper embodying the present invention.
Fig.2 is a front side view of the panty type diaper embodying the present invention.
Fig.3 is a cross-sectional view of an upper absorbent assembly.
Fig.4 is a Respective view showing upstanding cuffs.
Fig.5 is a schematic elevation view for illustrating a device for producing a main absorbent part.
Fig.6 is a schematic elevation view showing a device used for testing the volume of absorption on pressurization.

### Best Mode for Carrying out the Invention

A disposable panty type diaper, embodying the present invention, will now be described with reference to the drawings.

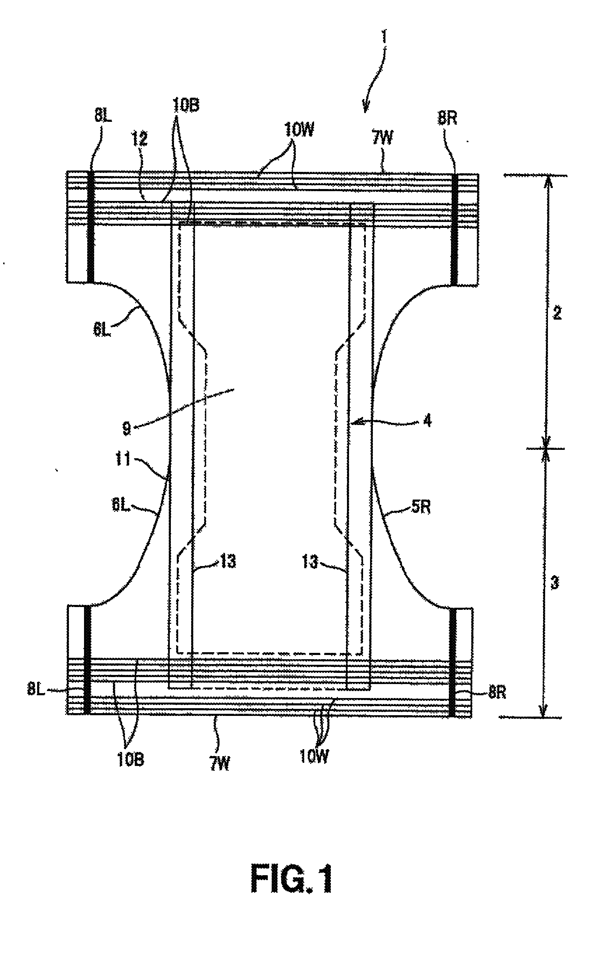

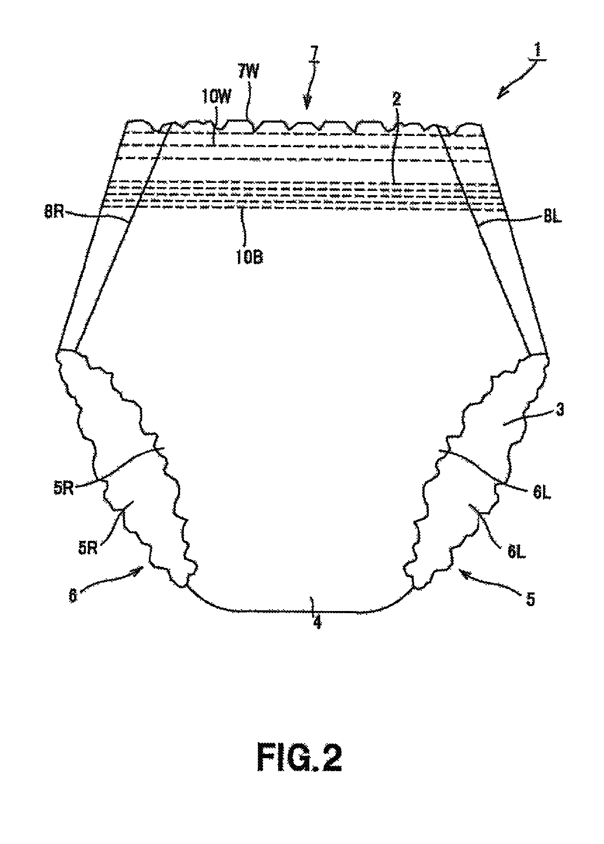

Referring to first Figs.1 and 2, a panty type diaper 1 is composed of a front body area 2 and a back body area 3 connecting as one to each other via a crotch cover area 4. On one longitudinal side of the diaper, right thigh swounding areas 5R, 5R connect as one to the front body area 2 and to the back body area 3. On the opposite longitudinal side of the diaper, left thigh surrounding areas 5L, 5L connect as one to the front body area 2 and to the back body area 3. On the short sides of the diaper, there are formed trunk surrounding areas 7W, 7W. Along the lateral sides of the front body area 2 and the back body area 3, there are provided junction areas 8R, 8L which are connected to each other. In forming the panty type diaper 1, the junction area 8R of the front body area 2 is abutted against the junction area 8R of the back body area 3 and, in this state, the junction areas 8R, 8R are unified together, by ultrasonic welding or thermal welding. On the other band, the junction area 8L of the front body area 2 is abutted against the junction area 8L of the back body area 3 and, in this state, the junction areas 8L, 8L are unified together by ultrasonic welding or thermal welding. With the panty type diaper 1, thus formed, a right thigh opening 5 is formed by the right thigh surrounding areas 5R, 5R of the front body area 2 and the back body area 3, whilst a left thigh opening 6 is formed by the left thigh surrounding areas 6L, 6L of the front body area 2 and the back body area 3. A trunk opening area 7 is formed by the trunk surrounding areas 7W, 7W of the front body area 2 and the back body area 3.

On the lateral sides of the trunk surrounding areas 7W, 7W of the front body area 2 and the back body area 3, there are provided one or plural elastic members 10W, each formed by an elastic yarn, for causing tight contact of the trunk opening area 7 around the trunk of the user, Below the elastic members 10W of the front body area 2, there are provided one or more elastic member 10B for causing tight contact of the front body area 2 and the back body area 3 around the belly part and the back part of the wearer.

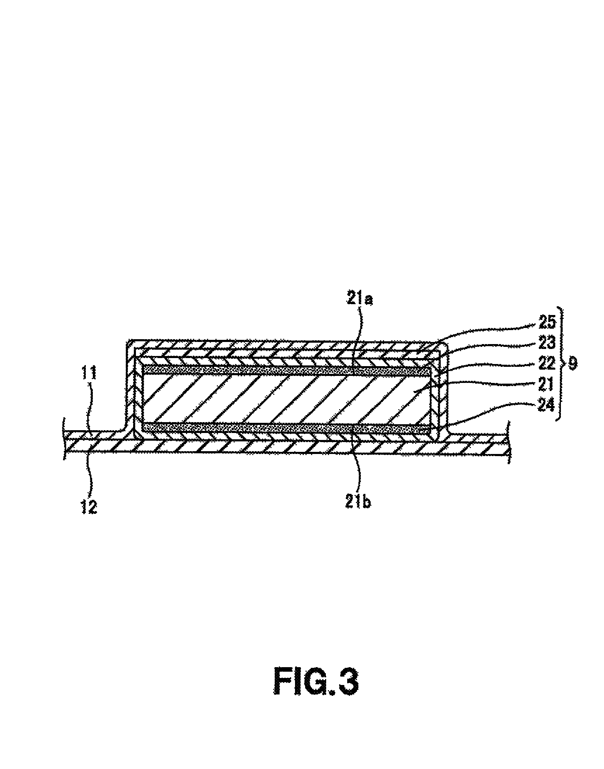

In a mid area of the inner surface of the diaper, lying between the front body area 2 and the back body area 3, there is provided an absorbent assembly 9 for absorbing the excremental matter. In the panty type diaper 1, made up of the front body area 2 and the back body area 3, connecting to each other as one via crotch cover area 4, the absorbent assembly 9 is sandwiched between a liquid permeable top sheet 11, lying on the inner side, and a liquid impermeable back sheet 12, lying on the outer side, as shown in Fig.3.

It should be noted that the top sheet 11 forms a surface directly contacted with the wearer's skin. Hence, it is necessary for the top sheet to be formed of a material exhibiting adaptability and flexibility and not exhibiting stimulating properties. For the top sheet 11, a porous or non-porous non-woven cloth or a non-porous plastics sheet, for example, is used. As fibers making up the non-woven cloth, regenerated fibers, such as rayon or cupra, or natural fibers, such as cotton, may be used, besides synthetic fibers, including olefinic fibers, such as polyethylene or polypropylene, or polyester-or polyamide-based fibers. The non-woven cloth is prepared by any of a variety of processing methods, such as spunlace method, a spunbond method, a thermal bond method, a melt blown method or a needle punch method.

The back sheet 12 is used for preventing the liquid, such as moisture, taken up by the absorbent assembly 9, from leaking to outside, and for preventing contamination e.g. of the apparel of the wearer. Thus, it is necessary for the back sheet to be liquid impermeable. Consequently, for the back sheet 12, a liquid impermeable sheet, such as polyethylene sheet, a water-shielding sheet, exhibiting permeability to humidity from the viewpoint of preventing stuffiness, or a composite sheet, made up by a non-woven cloth and a water-proofing sheet, may be used.

The top sheet 11 and the back sheet 12, described above, are unified together by ultrasonic or thermal welding, at the rim portions thereof, with the aforementioned absorbent assembly 9 in-between,

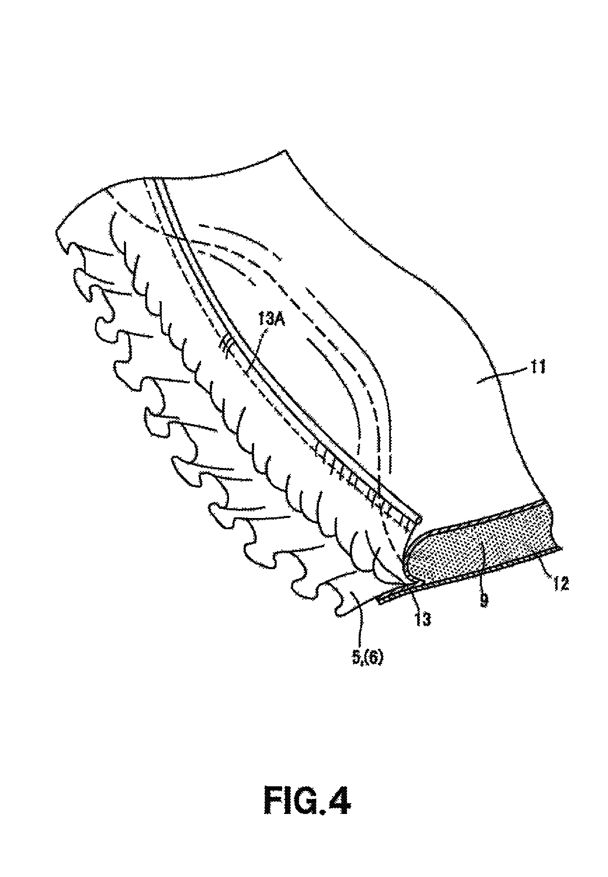

On each lateral side of the absorbent assembly 9, sandwiched between the top sheet 11 and the back sheet 12, there is provided an upstanding cuff 13 for preventing the wearer's excremental matter from leaking sideways via right thigh openings 5 and left thigh openings 6, as shown in Fig.4. Specifically, the upstanding cuffs 13 are provided to the top sheet 11 on both lateral side edges of the absorbent assembly 9 for extending along the right thigh surrounding areas 5R 5R and the left thigh surrounding areas 5L, 5L, as shown in Fig.1. The upstanding cuffs 13 are mounted upright so that the foremost parts thereof are directed towards the center of the absorbent assembly 9, as shown in Fig.4. The foremost parts of the upstanding cuffs 13 are provided with elastic members 13A adapted to be extended and contracted along with the right thigh opening 5 and the left thigh opening 6. When the diaper is worn, the upstanding cuffs 13 are kept in tight contact with the vicinity of the roots of the thighs of the wearer to prevent moisture, such as excretion, from leaking to outside. The upstanding cuffs 13 are formed of a material which is the same as that used for the top sheet 11 and the back sheet 12, for example, and are attached in position by, for example, ultrasonic welding or thermal welding. The upstanding cuffs 13 may, of course, be mounted as one with the top sheet 11 or the back sheet 12, using lateral side edges of these sheets 11 of 12,

Referring to Fig.3, the absorbent assembly 9 is prepared by wrapping a main absorbent part 21, formed of a highly absorbent polymer and absorbent fibers, with a crepe paper sheet 22, by bonding the major surface of the absorbent assembly 9 towards the top sheet 11 to the crepe paper sheet 22 with a first hot melt adhesive 23, by bonding the major surface of the absorbent assembly 9 towards the bottom sheet 12 to the crepe paper sheet 22 with a second hot melt adhesive 24, and by having a second sheet 25 interposed between the top sheet 11 and the crepe paper sheet 22.

The main absorbent part 21 is farmed by molding the highly absorbent polymer and absorbent fibers together. As the highly absorbent polymer, carboxyl methyl cellulose, polyacrylic acids, salts thereof, cross-linked acrylate polymers, starch-acrylic acid graft copolymers, hydrolyzates of starch- acrylonitrile graft copolymers, cross-linked polyoxyethylene, cross-linked carboxy methyl cellulose, partially cross-linked water-swollen polymers of polyethylene oxide or polyacrylamide, or an isobutylene- maleic acid copolymers, may be used. These materials may be added by a blocking inhibitor for suppressing the blocking otherwise caused by a product taking up the moisture. The highly absorbent polymer may be in the form of a powder, a particle, a granule, a pellet, a sol, a suspension, a gel, a film or a non-Woven cloth. Of these, the powder form of the highly absorbent polymer is most preferred.

As the absorbent fibers, used for the main absorbent part 21, cellulose fibers, obtained from wood, such as chemical pulp or dissolved pulp, or artificial cellulose fibers, such as rayon or acetate, may be used. Here, the pulp produced from needle-leaved trees, with longer fiber lengths, is preferred to the pulp obtained from broad-leaved trees, even though the latter is satisfactory in functions and costs.

The main absorbent part 21 may further be added by an adhesive, a diffusing agent, a deodorant or absorbent fibers.

With the main absorbent part 21, described above, the highly absorbent polymer is contained in an amount not less than 40 wt% and preferably in an amount of 40 to 80 wt% and more preferably in an amount of approximately 70 wt%, based on the total weight of the highly absorbent polymer and the absorbent fibers. That is, the proportion of the highly absorbent polymer in its entirety is increased to reduce the thickness of the ultimate product. As the highly absorbent polymer, used here, such polymer capable of absorbing larger quantities of the liquid under a pressurized condition, that is, the polymer having larger amounts of absorption on pressurization, is preferred. With the main absorbent part 21, the proportion of the highly absorbent polymer is higher, with the weight per unit area of the highly absorbent polymer being higher, If the weight per unit area of the highly absorbent polymer is higher, the highly absorbent polymer is in a compacted state. If the gel strength of the polymer is low, with the highly absorbent polymer becoming collapsed under a high load condition, the polymer is more compact and become almost completely impermeable to the liquid. For this reason, a highly absorbent polymer with the higher gel strength is used. Since there is no definite expression for the gel strength, it is here defined by stating that the larger the amount of liquid absorption of a highly absorbent polymer under a pressurized condition, the higher is the gel strength of the polymer.

The lower limit of the content of the highly absorbent polymer is set to 40% because the desired absorptive power may not be obtained if the amount of the polymer is less than this limit value. On the other hand, the upper limit of the content of the highly absorbent polymer is set to 80% because the desired absorptive power may not be obtained under the increased packing density of the structure and the gel blocking if the amount of the polymer is more than this limit value.

The above-described main absorbent part 21 may be produced by molding the absorbent fibers and the highly absorbent polymer in a state of a mixture or in a state in which the absorbent fibers and the highly absorbent polymer will form a superficial layer, with the pressure being applied during or after the molding for reducing the thickness. This pressurizing proms, aimed to reduce the thickness, is preferably carried out so that the weight per unit area and the thickness of the main absorbent part 21 will be not less than 300 gsm and not more than 2 mm, respectively, and so that the density thereof will be not less than 150 kg/m³. Most preferably, the thickness and the density of the main absorbent part are 1.0 to 1.8 mm and not less than 300 kg/m³, respectively.

Meanwhile, the thickness of the main absorbent part 21 in a state it is not pressurized for reducing its thickness is on the order of 2.5 to 3.5 mm.

Even though the main absorbent part 21 is increased in density in this manner, its absorption performance may be equivalent to that of the conventional product, not reduced in thickness, owing to the aforementioned properties of the highly absorbent polymer.

Several parameters of the highly absorbent polymer, actually used, are shown in Table 1.

**Table 1**

| | conventional | highly absorbent polymer used in embodiments of the invention |
|---|---|---|
| amount of absorption (g/g) | 53.0 | 52.0 |
| amount of water retention (g/g) | 34.0 | 32.0 |
| rate of absorption | 42.0 | 39.0 |
| amount of water absorption on pressurization (0.3 PSI) | 33.0 | 34.0 |
| grain size distribution 850 µm | 0.0 | 0.0 |
| 500 µm | 8.9 | 12.2 |
| 250 µm | 73.5 | 75.7 |
| 180 µm | 11.7 | 8.8 |
| 106 µm | 5.6 | 2.4 |
| pass | 0.3 | 0.9 |

The amount of liquid absorption was measured in the following manner. A 0.3 g sample of the main absorbent part 21 was charged into a nylon tea bag (pouch) which was then immersed in physiological saline (a 0.9% aqueous solution of NaCl). After ten minutes, the tea bag was completely Uplifted from the physiological saline and kept suspended for ten minutes to free the tea bag of water. After removal of water, the weight of the tea bag, containing the sample, was measured, and the weight of the sample and the tea bag was subtracted from the measured weight value. The difference obtained was assumed to be the absorbed amount at ambient pressure,

The amount of water retention was measured in the following manner. A sample of the main absorbent part 21 was charged into a nylon tea bag which was then immersed in 0.9% physiological saline. The tea bag-sample assembly was allowed to be swollen for 30 minutes and dehydrated by centrifugation for three minutes at 1500 rpm. The weight of the tea bag and the sample was subtracted from the weight of the dyhydrated tea bag-sample assembly and the resulting difference was divided by the sample weight to give a measure of the amount of water retention.

The rate of absorption was measured in the following manner. 1g of a sample of the main absorbent part 21 was evenly sprinkled in a Petri dish 90 mm across. On this sample was poured the artificial urine and the time until complete absorption was measured and used as the rate of absorption.

The grain size distribution was measured in the following manner, 10 g of a sample of the main absorbent part 21 were sifted through sieves of 850 µm, 500 µm, 250 µm, 180 µm and 106 µm. The weights of the sample left in the sieves were measured and expressed in percentages, as the loss resulting from step-up of the sieves was taken into account The sieving time was set to one minute. It is seen from Table 1 that, with the highly absorbent polymer of the present invention, the amount of the small-sized products, less than 180 µm in diameter, has been decreased

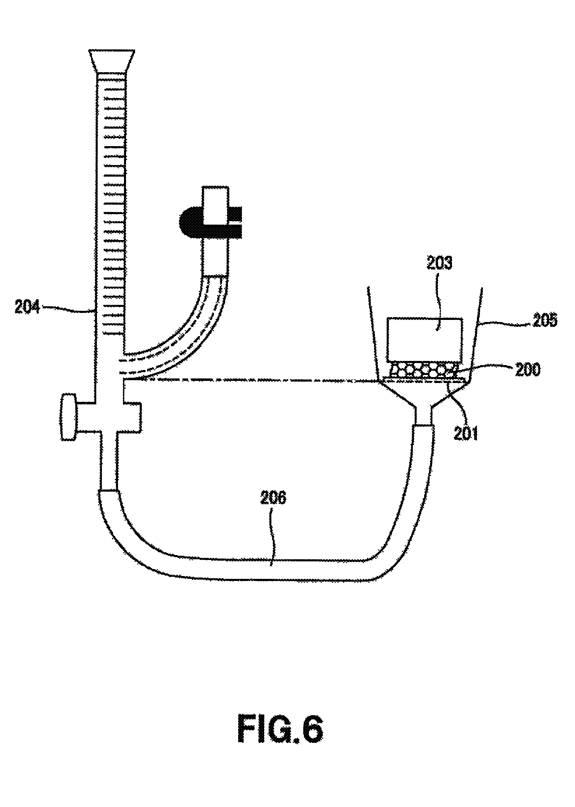

The amount of water absorption on pressurization was measured using an apparatus shown in Fig.6. Specifically, 0.2 g of a sample 200 of the main absorbent part 21 was weighed and evenly sprinkled within a circle 40 mm in diameter on a filter paper sheet 201 of 50 mm in diameter. A load of 20 g/cm² was set thereon using a weight 203. The four sides of the weight 203 were secured to the filter paper sheet 201 by a cellophane tape 1.5 mm width. A measurement device composed of a burette 204 and a funnel 205, a lower opening of which is coupled via a conduit 206 to a discharge opening of the burette 204, was provided. The sample 200 was quietly placed within the funnel 205, with the sample clamped by the weight 202 against the filter paper sheet 201, with the filter paper sheet 201 facing downwards. In this state, the amount absorbed by the main absorbent part within 60 minutes was measured. The amount absorbed by the filter paper sheet by itself, as a blank, was similarly measured. The amount absorbed after 60 minutes was re-calculated for 1.0g of the sample and the so re-calculated value was used as a measure of the amount of water absorption on pressurization. It is seen from Table 1 that, with the highly absorbent polymer of the present invention, the amount of absorption on pressurization has been increased beyond the amount obtained with the state-of-the-art polymer, thus testifying to the gel strength of the highly absorbent polymer of the present invention higher than that of the state-of-the-art polymer.

The main absorbent part 21 has a substantially planar surface which is subjected to embossing. By this embossing, the highly absorbent polymer is pressed down by the absorbent fibers, on the surface of the main absorbent part 21, for thereby positively anchoring the highly absorbent polymer in place. In the related art, a projection is formed on the main absorbent part 21 for extending along the inguinal region of the wearer, According to the present invention, the main absorbent part 21 has a substantially planar surface for uniformly performing the embossing.

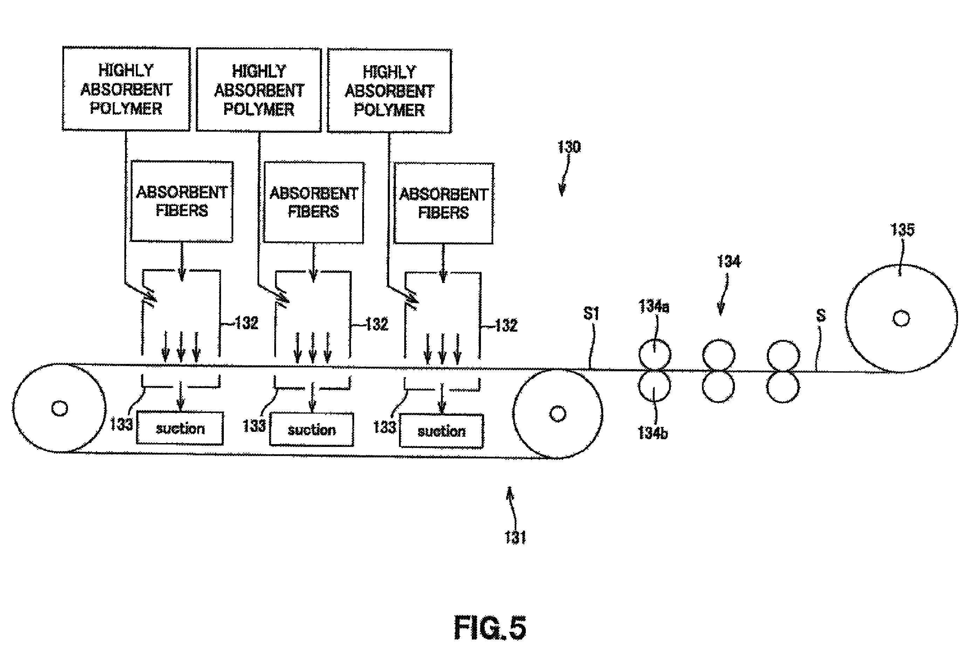

The method for producing the main absorbent part 21, described above, will now be described. Although there may be a variety of methods for producing the main absorbent part 21, an air laying method is here used from the viewpoint of homogeneously mixing the absorbent fibers and the highly absorbent polymer and for improving the manufacture efficiency. With this air laying method, both the absorbent fibers and the highly absorbent polymer are collected and heaped up by an air stream so as to be thereby shaped together. Referring to Fig.5, a device for manufacturing the main absorbent part 130 is made up by a belt conveyor type air laying section, a set of heating/ pressurizing rolls 134 and a take-up roll 135. The air laying unit includes an endless belt conveyor 131, having an aerating structure, a set of sprinkling chutes 132, 132, ..., and a plural number of suction sections 133, 133, ···. The sprinkling chutes are arranged on the upper surface of the endless belt conveyor 131 as these chutes are spaced apart from one another along the transport direction, The suction sections are arranged on the back side of the conveyor 131 in register with the sprinkling chutes 132, 132, ···. The set of heating/ pressurizing rolls 134 pressurize the heaped matter discharged from the air lay section to reduce the thickness thereof while anchoring the fibers to one another. The take-up roll 135 takes up a sheet S of the main absorbent part 21 pressurized and reduced in thickness.

In the manufacturing device 130, the absorbent fibers and the highly absorbent polymer are entrained in respective air steams and thereby injected onto the same sprinkling chutes 132 where the fibers and the polymer are opened and mixed together. The fibers and the polymer are also heaped and molded in situ as a web S1 on the conveyor 131 by suction from the back side of the conveyor 131. The web S1, thus heaped and molded in situ, is transferred from the conveyor 131 to the set of heating/ pressurizing rolls 134, where the web is clamped between heating/ pressurizing rolls 134a, 134b. The web is pressurized by these rolls and reduced in thickness, while the fibers of the web are anchored to one another to form an air-laid absorbent sheet S. The sheet S has its surface processed with embossing and is taken up on the take-up roll 135, This take-up roll 135 is brought into a production line for the panty type diaper 1 where the sheet S is unreeled and cat to preset shape in a well-known manner.

With the use of the manufacturing device 130, the main absorbent part 21, the highly absorbent polymer component of which accounts for 40 wt% or more based on the total weight of the absorbed fibers and the highly absorbent polymer, may be produced. Moreover, by adjusting the pressure applied by the heating/pressurizing rolls 134a, 134b, the main absorbent part 21, with the weight per unit area not less than 300 gsm, the thickness less than 2 mm and with the density not less than 150 kg/m³, may be produced. Additionally, the main absorbent part 21 which is more preferred, that is, the main absorbent part having a thickness of 1.0 to 1.8 mm and a density not less than 300 kg/m³, may be produced.

The main absorbent part 21, manufactured by the above-described manufacturing device 130, is wrapped up by a crepe paper sheet 22, composed of cotton-like pulp and exhibiting certain toughness, as shown in Fig.3. It is noted that one of the major surfaces 21a of the main absorbent part 21 towards the top sheet 11 is unified to the crepe paper sheet 22 with the first hot melt adhesive 23. The first hot melt adhesive 23 is used for improving the anchoring of the highly absorbent polymer of the main absorbent part 21 and for improving the gel strength. It is adapted to seep into the main absorbent part 21. A delayed crystallizing adhesive, specifically a styrene-isoprene-styrene based adhesive, is used. The opposite major surface 21b of the main absorbent part 21 towards the back sheet 12 is unified to the crepe paper sheet 22 with the second hot melt adhesive 24. This second hot melt adhesive 24 is used for anchoring the highly absorbent polymer to the absorbent fibers to prevent the gel from coming apart or from becoming cracked after the highly absorbent polymer has absorbed e.g. the urine and has become gelated, For the second hot melt adhesive, a water-proofed adhesive, specifically, a styrene-ethylene-butylene-styrene based adhesive, is used.

Meanwhile, with the conventional product, the same adhesive, specifically, an olefinic adhesive, has been used for the first hot melt adhesive 23 and for the second hot melt adhesive 24.

The second sheet 25, interposed between the top sheet 11 and the crepe paper sheet 22, is used for diffusing e.g, the urine, permeated from the top sheet 11, before the urine is introduced into the main absorbent part 21, for allowing the urine to seep extensively into the main absorbent part 21. Hence, the second sheet is formed of a material exhibiting the high porosity, that is, a material which is thicker and higher in bulkiness. On the other hand, since the second sheet 25 has also been press-worked, it is preferably of a material having high post-pressing restoration properties and the high cushioning performance. As the second sheet 25, PE/PET fibers, formed from polyethylene (PE) and polypropyrene (PP), have so far been used. Here, PE/PET fibers, formed from polyethylene (PE) and polyethylene terephthalate (PET), are used, because this material is higher in specific gravity than PE and may be higher in porosity, while being superior in post-possing restoration properties. More specifically, PE/PET fibers, formed of PET as a core material and PE formed therearound, or PE/PET fibers, formed of PE as a core material and PET formed therearound, are used. With the use of the second sheet 25, formed in part of PET, namely PE/ PET fibers, the urine, for example, may be evidently taken up by the capillary phenomenon and diffused into the main absorbent part 21.

In the second sheet 25, hollow PET fibers, having a hollow core part, may also be used as a component for increasing the porosity and post-pressing restoration performance.

As for the size, the fibers of 5.6 dt or 4.4 dt in thickness have so far been used. Here, the fibers thicker in thickness than the conventional fibers are used, with a view to further raising the porosity and post-pressing restoration performance and to achieving more efficient absorption of the urine by the capillary phenomenon. Specifically, the PET fibers with the size of 6.4 dt, and the hollow PET fibers with the size of 6.0 dt, are used.

The second sheet 25 has a weight per unit area of 40 gsm, which is of the same order of magnitude as that of the conventional product.

The absorbent assembly 9 uses the main absorbent part 21, containing the highly absorbent polymer in a proportion higher than in the conventional practice, in order to reduce the thickness. Moreover the absorbent assembly 9 has its surface embossed, and the highly absorbent polymer is anchored to the absorbent fibers, using the first hot melt adhesive 23, for preventing the absorptive power of the main absorbent part 21 from being lowered, In addition, PET fibers or hollow PET fibers are used for the second sheet 25 of the absorbent assembly 9 to cause e.g. the urine to be contacted over a wide area with the main absorbent part 21 to prevent the global lowering of the absorptive power.

The panty type diaper 1, described above, is carried about by a user in a collapsed and compression-pressed state. Since the absorbent assembly 9, reduced in thickness as described above, is used, the overall thickness in the collapsed state of the panty type diaper may be reduced. Hence, the panty type diaper may readily be carried about despite the fact that the diaper has the absorptive power equivalent to or exceeding the absorptive power of the conventional product.

The present invention has so far been described with reference to a case of the panty type diaper 1. However, the present invention is not limited to this configuration and may also be applied to a tape type diaper 1, to a physiological napkin and to an incontinence pad.

Although the present invention has so far been explained with reference to the preferred embodiments, shown in the accompanying drawings, the present invention is not limited to the particular configurations of these embodiments. It will be appreciated that the present invention may encompass various changes or corrections such as may readily be arrived at by those skilled in the art within the scope and the principle of the invention.

### Industrial Applicability

According to the present invention, the main absorbent part is made up of absorbent fibers and the highly absorbent polymer, the weight ratio of which to the total weight of the absorbent fibers and the highly absorbent polymer amounts to not less than 40 wt%, and the main absorbent part, thus formed, is pressurized to reduce the overall thickness. In addition, the liquid permeable sheet, layered on the main absorbent part, is formed, at least in part, of polyethylene terephthalate, exhibiting the high cushioning performance, for diffusing the liquid and for enlarging the area over which the liquid is intruded into the main absorbent part, for thereby preventing the absorptive power from being lowered.

## Claims

1. An absorbent assembly comprising;
a main absorbent part composed of a highly absorbent polymer and absorbent fibers, a crepe paper sheet wrapped around said main absorbent part, the major surfaces of said main absorbent part being hot-melt bonded to said crepe paper sheet, and a liquid permeable sheet arranged on a portion of said crepe paper sheet wrapped around one of the major surfaces of said main absorbed part; wherein
the weight ratio of said highly absorbent polymer to the total weight of said highly absorbent polymer and absorbent fibers of said main absorbent pan is set to 40 wt% or higher, said main absorbent part being pressurized and reduced in thickness,
said liquid permeable sheet being formed, at least in part, of polyethylene terephthalate as a component thereof.

2. The absorbent assembly according to claim 1 wherein said liquid permeable sheet is also formed, in part, of hollow polyethylene terephthalate.

3. The absorbent assembly according to claim 1 or 2 wherein a delayed crystallization adhesive is used for hot melt bonding between said one major surface of said main absorbent part and said crepe paper sheet, and a water-proofing adhesive is used for hot-melt bonding between the opposite major surface of said main absorbent part and said crepe paper sheet.

4. The absorbent assembly according to claim 3 wherein the surface of said main absorbent part is substantially planar and has been processed with embossing.

5. The absorbent assembly according to claim 1 wherein the weight per unit area and the density of said highly absorbent polymer are not less than 300 gsm and not less than 150 kg/m³, respectively, and the overall thickness of said highly absorbent polymer is not more than 2 mm.

6. The absorbent assembly according to claim 1 wherein the overall thickness of said main absorbent part is 1.0 to 1.8 mm and the density of said highly absorbent polymer is not less than 300 kg/m³.

7. A diaper wherein
lateral side edges of a front body area and a back body area are unified together to form a trunk opening area and left and right thigh opening areas, with an absorbent assembly being formed at a center area,
said absorbent assembly being arranged between a liquid permeable top sheet and a liquid impermeable back sheet,
said absorbent assembly including
a main absorbent part composed of a highly absorbent polymer and absorbent fibers, a crepe paper sheet wrapped around said main absorbent part, the major surfaces of said main absorbent part being hot-melt bonded to said crepe paper sheet, and a liquid permeable second sheet arranged between said top sheet and the crepe paper sheet, wherein
the weight ratio of said highly absorbent polymer to the total weight of said highly absorbent polymer and absorbent fibers of said main absorbent part is set to 40 wt% or higher, said main absorbent part being pressurized and reduced in thickness,
said liquid permeable sheet being formed at least in part of polyethylene terephthalate as a component thereof.

8. An absorbent product comprising:
an absorbent assembly arranged between a liquid permeable top sheet and a liquid impermeable back sheet;
said absorbent assembly being arranged between the liquid permeable top sheet and the liquid impermeable back sheet,
said absorbent assembly including
a main absorbent part composed of a highly absorbent polymer and absorbent fibers, a crepe paper sheet wrapped around said main absorbent part, the major surfaces of said main absorbent pan being hot-melt bonded to said crepe paper sheet, and a liquid permeable second sheet arranged between said top sheet and the crepe paper sheet, wherein
the weight ratio of said highly absorbent polymer to the total weight of said highly absorbent polymer and absorbent fibers of said main absorbent part is set to 40 wt% or higher, said main absorbent part being pressurized and reduced in thickness,
said second sheet being formed, at least in part, of polyethylene terephthalate as a component thereof,
